# EUROPEAN PATENT SPECIFICATION

(11) **EP 4 402 286 B1**
(45) Date of publication and mention of the grant of the patent: **22.04.2026**
(21) Application number: 22870546.3
(22) Date of filing: 09.09.2022
(51) Int. Cl.: C12Q 1/6858, C12Q 1/6855, C12Q 1/6811, C12Q 1/6804

(54) **METHODS FOR IDENTIFYING PROTEIN CODING SEQUENCES USING DNA BARCODES**
VERFAHREN ZUR IDENTIFIZIERUNG VON PROTEINKODIERENDEN SEQUENZEN MITTELS DNA-STRICHCODES
PROCÉDÉS D'IDENTIFICATION DE SÉQUENCES DE CODAGE DE PROTÉINES À L'AIDE DE CODES-BARRES D'ADN

(30) Priority: 16.09.2021 US 202163244957 P
(43) Date of publication of application: 24.07.2024
(73) Proprietor: A-Alpha Bio, Inc., Seattle, Washington 98195 (US)
(72) Inventor: YOUNGER, David, Seattle, Washington 98195 (US); LOPEZ, Randolph, Seattle, Washington 98195 (US); EMERSON, Ryan, Seattle, Washington 98195 (US)
(74) Representative: Eisenführ Speiser
(86) International application number: PCT/US2022/043139
(87) International publication number: WO 2023/043685

(56) References cited:
- WO-A1-2019/046564
- WO-A2-2015/085079
- US-A1- 2008 287 320
- US-A1- 2020 325 526
- GUERNET ALEXIS ET AL: "CRISPR-Barcoding for Intratumor Genetic Heterogeneity Modeling and Functional Analysis of Oncogenic Driver Mutations", MOLECULAR CELL, ELSEVIER, AMSTERDAM, NL, vol. 63, no. 3, 21 July 2016 (2016-07-21), pages 526 - 538, XP029675840, ISSN: 1097-2765, DOI: 10.1016/J.MOLCEL.2016.06.017

## Description

### RELATED APPLICATIONS

This application claims priority to U.S. Ser. No. 63/244,957 filed on September 16, 2021.

### BACKGROUND INFORMATION

Much of molecular biology and biochemistry is accomplished via the construction of mutagenic libraries: pluralities of partially randomized sequences in which an initial protein-coding sequence (referred to as a wild-type, reference, or parental sequence, typically referred to herein as a "reference" sequence or "reference" protein) is mutated at one or more positions to generate a large number of mutational variants for expression and characterization in later experiments. An essential part of this process is the mapping from deoxyribonucleic acid (DNA), which can be measured experimentally from such libraries, to the protein species expressed by each DNA molecule. Such mapping can be accomplished by sequencing the protein-coding region of the DNA library directly, but this approach typically requires long-read sequencing, *i.e.,* sequencing reads of sufficient length to directly observe the entire DNA region of interest.

Another approach is to incorporate a user-designed synthetic DNA barcode on the protein-coding DNA molecule that may be sequenced to identify the mutated region of interest, but such barcodes comprising random DNA sequence makes them incompatible with maintaining a fully defined open reading frame (ORF) and must thus be placed outside the protein-coding region bearing the actual mutational variants. The identifying synthetic DNA barcode must be in some way associated with the mutated region of interest within the protein-coding sequence, with a known relationship confirming that the identifying barcode and mutated region of interest are on the same molecule. There are two immediately apparent options to establish this relationship. First, the entire sequence spanning both non-coding user-designated synthetic DNA barcode and protein-coding mutated region of interest may be synthesized as a single molecule such that the relationship between identifying synthetic DNA barcode and the region of interest is established in synthesis with no need to map experimentally. However, fully-custom oligonucleotide synthesis of molecules of such lengths is prohibitively expensive for large-scale high-throughput library-based experiments. Second, one may ligate a library of random DNA barcodes onto a library of mutated region-of-interest DNA sequences or use primers with barcodes in their overhangs to polymerase chain reaction (PCR) a library of random DNA barcodes onto a library of mutated region-of-interest DNA sequences, generating a random combination of synthetic DNA barcodes and protein-coding regions. Once assembled, the DNA library can be sequenced from the non-coding user-designed synthetic DNA barcode, into the protein-coding segment of DNA, through the mutated region of interest in order to map the unique identifying DNA barcode to the unique mutated region of interest. However, this approach requires using a sequencing platform that produces relatively long sequencing reads (*e.g*., Pacific Biosciences' single-molecule real-time sequencing or Oxford Nanopore Technologies' nanopore sequencing which at present routinely produce reads exceeding 10 kilobases (kb)), reads of which are known to those of ordinary skill in the art to be of lower quality and/or higher cost than other high-throughput sequencing methods (*e.g*., Illumina's NovaSeq, HiSeq, NextSeq, and MiSeq platforms) which utilize shorter reads, are more economical, and are more accurate. In addition, in some implementations of such mutagenic libraries, one mutational variant of the region of interest may be nearly identical to another mutational variant of the region of interest, possibly differing by only a single base pair substitution. Such modest differences between mutational variants of a mutagenic library are prone to being disguised by the relatively high error rate of currently available long sequencing reads and may thus render two relatively close mutational variants unresolvable via sequencing.

Accordingly, there exists a need in the art for a method to resolve the relationship between unique user-designed synthetic DNA barcodes and a protein-coding mutated region of interest with enhanced accuracy that is amenable to short-read sequencing platforms (*e.g*., Illumina sequencing platforms). In addition, there exists a need to introduce increased sequence divergence between mutational variants of a region of interest in order to enhance the resolvability of mutational variants within a mutagenic library when error-prone long-read sequencing platforms (*e.g*., PacBio or Oxford Nanopore Technologies) are used. The methods disclosed herein meet that need.

WO 2015/085079 discloses that the identification of binding moieties capable of selectively interacting with one or more target antigens is of scientific, medical, and commercial value. Disclosed herein are methods and compositions for the identification, labeling, and/or retrieval of cognate binding moieties.

XP029675840 (GUERNET ALEXIS ET AL: "CRISPR-Barcoding for Intratumor Genetic Heterogeneity Modeling and Functional Analysis of Oncogenic Driver Mutations") refers to CRISPR/Cas9 technology to genetically label and trace tumor cells within a mass population through insertion of a series of point mutations at a specific genomic location. The effects of a particular experimental condition on the fraction of barcoded cells can be assessed by qPCR or deep sequencing.

WO 2019/046564 A1 discloses compositions and methods for high-through-put screening a plurality of transposases to identify rare mutations that affect desired fea-tures of the transposase. Compositions of the disclosures are provided that facilitate the high-throughput screening methods of the disclosure.

### BRIEF DESCRIPTION OF THE DRAWINGS

The accompanying drawings, which are incorporated in and constitute a part of the specification, illustrate one or more embodiments and, together with the description, explain these embodiments. The accompanying drawings have not necessarily been drawn to scale. Any values and/or dimensions illustrated in the accompanying graphs and figures are for illustration purposes only and may or may not represent actual or preferred values or dimensions. Where applicable, some or all features may not be illustrated to assist in the description of underlying features. In the drawings:

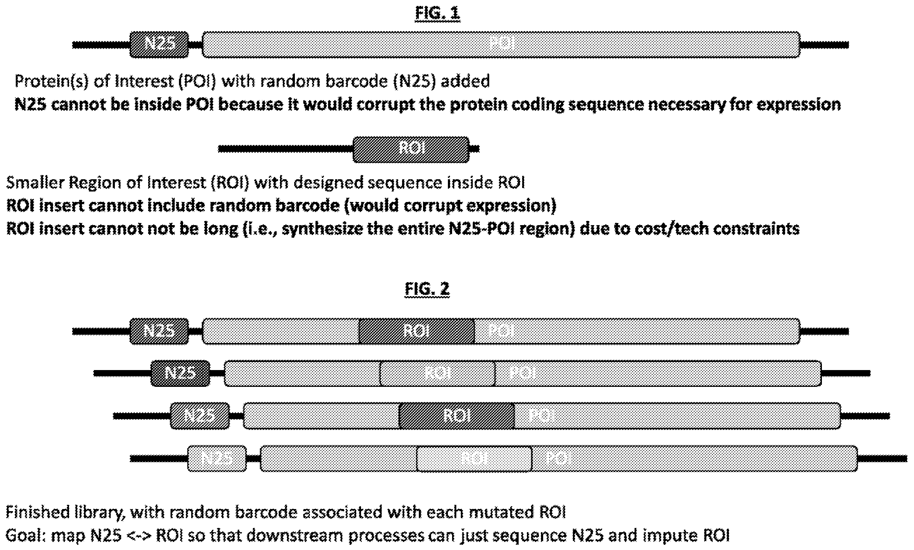

FIG. 1 is a depiction of an exemplary polynucleotide sequence encoding a protein of interest (POI) with an exemplary "random" 25-nucleotide barcode (N25) added upstream (5') of the open reading frame (ORF) of the POI and a region of interest (ROI) (having a designed nucleotide sequence length shorter than that of the POI) which represents user-designated variants (mutants) to be introduced to the sequence encoding the protein of interest. The "random" barcode, in this figure being 25 nucleotides in length (*i.e.,* N25) is one that can be of any nucleotide sequence and length (*e.g.,* any of about 10, 15, 20, 25, 30 or more nucleotides) that could be designed or randomly generated ("random"), provided the sequence thereof does not interfere with expression of the POI and/or ROI. For instance, the random bar code (N25) cannot be positioned within the coding sequence of the POI as doing so would corrupt expression of the POI.
FIG. 2 is a depiction of an exemplary library of polynucleotide sequences, each encoding a POI, each comprising a unique region of interest (ROI), and each including a unique random 25-nucleotide barcode (N25) added upstream of the open reading frame. Polynucleotides comprising the ROI can then be imputed by sequencing only the N25 sequence (random barcode).

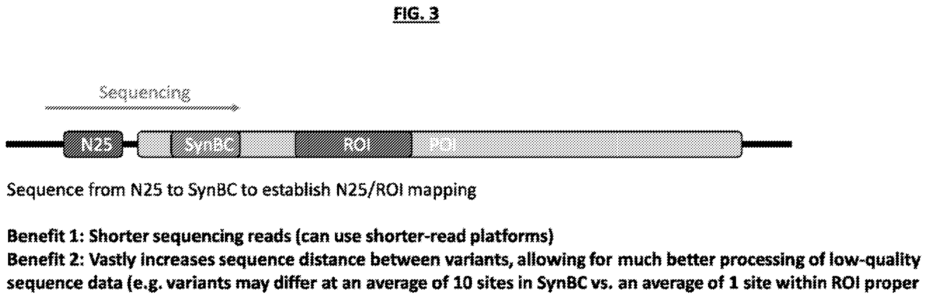

FIG. 3 is a depiction of a polynucleotide sequence of this disclosure encoding a protein of interest (POI) with an exemplary second barcode (*e.g*., a "random" or "randomly generated" 25-nucleotide barcode (N25)) added or positioned upstream (5') of the open reading frame (ORF) and a region of interest (ROI) which represents user-designated nucleotide sequence variants within the nucleotide sequence encoding the protein of interest (POI), wherein a unique synthetic barcode (SynBC) that introduces one or more silent mutations relative to a reference sequence coupled to the unique ROI has been introduced to the polynucleotide sequence, wherein the second barcode (*e.g.,* the N25 sequence) and the SynBC sequence can be sequenced by one of several commercially available short-read or long-read sequencing platforms to identify a particular POI comprising a particular ROI.
FIG. 4 presents the expected DNA sequence of all elements of a Site Saturation Mutagenesis (SSM) library covering exemplary RBD POIs.
FIG. 5 presents the expected DNA sequence of all elements of a Site Saturation Mutagenesis (SSM) library covering exemplary RBD POIs.
FIG. 6 presents the expected DNA sequence of all elements of a Site Saturation Mutagenesis (SSM) library covering exemplary RBD POIs.
FIG. 7 presents the expected DNA sequence of all elements of a Site Saturation Mutagenesis (SSM) library covering exemplary VEGF-A POIs.
FIG. 8 presents the expected DNA sequence of all elements of a Site Saturation Mutagenesis (SSM) library covering exemplary VEGF-A POIs.
FIG. 9 is a histogram plot representing sequencing reads of polynucleotides with and without SynBCs (codon-shuffling) binned by BLASTN score between the best match and the second-best match compared to expected POI sequences.
FIG. 10 is a histogram plot representing sequencing reads of polynucleotides with and without SynBCs (codon-shuffling) binned by Consensus score between the best match and the second-best match compared to expected POI sequences.

### SUMMARY OF THE DISCLOSURE

This disclosure provides polynucleotides, combinations of polynucleotides, and cells comprising the same, as well as methods for identifying protein-coding regions within polynucleotides (*e.g*., within a library of polynucleotides at least some of which encode different proteins of interest (POIs)) based on the identification of at least one "barcode" associated with each of said polynucleotides. In preferred embodiments, a particular POI comprising a particular region of interest ("ROI", a pre-designed region of the POI comprising at least one nucleotide sequence difference from a reference POI), at least one silent mutation providing a first barcode within the POI without changing the amino acid sequence encoded by the POI, and at least one second barcode having a "random" (*e.g*., non-protein coding) nucleotide sequence positioned upstream (5') and within a particular number of nucleotides or base pairs from the beginning of the POI, and especially from the first barcode (*e.g*., within 600 nucleotides or base pairs). Preferably, such a polynucleotide encoding a particular POI comprising a particular ROI can be identified by sequencing the first and second barcodes only, or in some embodiments only the second barcode. Other embodiments are also provided as will be apparent to those of ordinary skill in the art from this disclosure.

### DETAILED DESCRIPTION

The description set forth below in connection with the appended drawings is intended to be a description of various, illustrative embodiments of the disclosed subject matter. Specific features and functionalities are described in connection with each illustrative embodiment; however, it will be apparent to those skilled in the art that the disclosed embodiments may be practiced without each of those specific features and functionalities.

Provided are methods for resolving (*e.g*., determining) the relationship between unique user-designed and/or random (or randomized) synthetic DNA barcodes (first (SynBC) and second barcodes, respectively) and a protein-coding mutated region of interest (ROI) with enhanced accuracy that is amenable to short-read sequencing platforms. In addition, the methods introduce increased sequence divergence between mutational variants of a region of interest in order to enhance the resolvability of mutational variants within a mutagenic library when error-prone and/or costly long-read sequencing platforms are used. The methods described herein also provide for the use of only short-read sequencing platforms to identify polynucleotides encoding a particular protein of interest (POI) or region of interest (ROI) thereof, preferably by identifying only the first (have a designed sequence difference from a "reference" sequence (*e.g*., wild-type and/or parental POI) and second (having a "random" sequence) barcodes thereof, or only the second barcode thereof.

This disclosure provides polynucleotides, combinations of polynucleotides, and cells comprising the same, as well as methods for identifying protein-coding regions within polynucleotides (*e.g.*, within a library of polynucleotides at least some of which encode different POIs) based on the identification of at least one "barcode" associated with each of said polynucleotides. In some embodiments, this disclosure provides methods for pairing protein coding regions with one or more of such barcode(s) so that short-read sequencing can then be used to identify a polynucleotide or cell comprising a particular protein coding region. In some embodiments, this disclosure provides methods for pairing protein coding regions (*e.g.,* preferably encoding POIs) with one or more short barcodes (*e.g.,* the first and second barcodes), which then allows for the identification of coding regions by sequencing only the one or more short barcodes. In some embodiments, one barcode can be sequenced. In some embodiments, more than one barcode can be sequenced. In some embodiments, this disclosure provides methods for identifying protein coding regions with short-read sequencing (*i.e.,* less than about 600 nucleotides; using, *e.g*., short-read "next generation sequencing" ("NGS")) whereby a polynucleotide protein coding region is first paired with a short polynucleotide barcode into a single polynucleotide, and then identified as being paired with a particular protein-coding region using long-read sequencing (*i.e.,* greater than about 600 nucleotides; *e.g.,* long-read NGS), such that the polynucleotide can subsequently be identified by short-read sequencing (*e.g*., less than about 600 nucleotides, such as but not limited to about any of 100, 150, 200, 250, 300, 350, 400, 450, 500 or 550 nucleotides) of the one or more polynucleotide barcodes (in some preferred embodiments only the second (randomized) barcode) without necessarily sequencing the entire protein-coding region of the polynucleotide.

The polynucleotides disclosed herein comprise at least first and second barcodes. A "barcode" is a particular polynucleotide sequence contained within the polynucleotides of this disclosure. A first barcode (also referred as "synonymous" or "SynBC" or "codon-shuffling") is incorporated into the protein-coding region of a polynucleotide encoding a POI, and a second barcode (*i.e.,* "randomized" barcode) is incorporated outside the protein-coding region of the same polynucleotide. Preferably each member of a group of polynucleotides (*e.g*., a polynucleotide library) includes different first and second barcodes, but most preferably at least different second barcodes. The first barcode is "silent" relative to the POI encoded by a reference sequence (which is referred to in some embodiments herein as "SynBC"; *e.g.,* a wild-type (*e.g*., naturally-occurring) or parental polynucleotide (a "reference" polynucleotide) which may include one or more nucleotide differences from the wild-type polynucleotide that can be silent or non-silent. A "silent" mutation is one that includes at least one nucleotide substitution relative to the reference sequence and does not change the amino acid sequence of the POI encoded by the polynucleotide including the silent mutation. A non-silent mutation is at least one nucleotide difference relative to the reference sequence that results in at least one amino acid change to the POI encoded by the reference polynucleotide. The second barcode is "random" or "randomized" in that it does not necessarily have a particular pre-determined nucleotide sequence (although in some embodiments it can be pre-determined or designed) and does not encode or would not be used by a cell to produce a protein or interact with the nucleotide sequence encoding the POI such the expression and/or sequencing of either is hindered (*i.e.,* interfered with), but is unique relative to other second barcodes in polynucleotides of a polynucleotide library. This second barcode can be of any suitable length (*i.e.,* number of nucleotides or base pairs), such as any of about 100, 90, 80, 70, 60, 50, 40, 30, 25, 20, 15 or 10 nucleotides. In some preferred embodiments, the second barcode is a random non-protein encoding nucleotide sequence of 25 nucleotides or base pairs (*e.g.,* N25 as exemplified in Fig. 3). The second barcode could be of any suitable length, but is preferably at least about 10, 15, 20, 25, or 30 nucleotides in length. In some embodiments, the second barcode can be specifically designed or randomly generated using any techniques available to those of ordinary skill in the art.

In some embodiments, the nucleotide sequence encoding the region of interest may differ from the reference sequences (*e.g*., other members of a site-saturation mutagenesis library) by only a single base-pair (or nucleotide), and such single-base pair substitutions may be undetectable by long-read sequencing platforms due to the high error-rate of such platforms. The first barcode (SynBC) can be, and preferably is, included within the polynucleotide encoding the POI. In some embodiments, the first barcode (SynBC) can be placed within the polynucleotide sequence encoding the POI between the beginning of the open reading frame (ORF) and the polynucleotide sequence encoding a region of interest (ROI) of the POI. In other embodiments, the first barcode (SynBC) can be placed within the polynucleotide sequence encoding the POI between the polynucleotide sequence encoding a region of interest (ROI) of the POI and the end of the open reading frame (ORF) (see, *e.g.,* Fig. 3). In other embodiments, the first barcode (SynBC) can be placed either partially or completely within the region of interest (ROI) of the POI. The first barcode (SynBC, incorporated into a protein-coding region of the polynucleotides) may be any number of nucleotides. The first barcode (SynBC) includes at least one nucleotide difference from a reference polynucleotide (*e.g*., wild-type, parental or reference polynucleotide) encoding the POI, resulting in no amino acid change to the protein-coding region (*i.e.,* relative to the reference (*e.g.,* parental or "wild-type" (*e.g.,* naturally-occurring) protein-coding region). In preferred embodiments, the first barcode comprises one to 100 (*e.g*., about or exactly any of 1, 5, 10, 15, 20, 25, 30, 35, 40, 45, 50, 55, 60, 65, 70, 75, 80, 85, 90, 95 or 100) nucleotide differences from a wild-type (*e.g*., naturally-occurring), parental, reference, or other nucleotide sequence of the protein-coding region (which can be collectively referred to herein by the term "reference" sequence). The protein-coding region can comprise hundreds or thousands of nucleotides (*e.g*., multiple kilobases (kb)) and can, and in some preferred embodiments does, include one or more nucleotide mutations (*e.g*., differences) relative to the reference nucleotide sequence, that introduce a change in the amino acid sequence of the POI (*i.e.,* a "non-silent" mutation).

A wild-type version of the protein-coding region can be a version of the POI that is found in nature and can serve as a reference sequence. A parental or reference protein-coding region can also be one with which the mutated version shares significant similarity, and/or was derived, or as may be otherwise understood by those of ordinary skill to be similar but different from the protein-coding region of the polynucleotide of the polynucleotide library. The at least one nucleotide difference of the first barcode does not change the amino acid sequence of the POI but can be used to distinguish one first barcode in one polynucleotide from another first barcode in another polynucleotide regardless of the presence or number of differences in the protein-coding region of the two polynucleotides resulting in non-silent mutations. Typically, the first barcode(s) is specifically designed to have a particular sequence and can be synthesized as an oligonucleotide and incorporated into a polynucleotide encoding the POI using standard techniques. In some preferred embodiments, the ROI (which may contain one or more non-silent mutations) and the first barcode(s) can be synthesized as a single oligonucleotide and incorporated into a polynucleotide encoding the second (randomized) barcode(s) using standard techniques. In some embodiments, the first barcode(s) and second (randomized) barcode(s) can be synthesized as a single oligonucleotide and incorporated into a polynucleotide encoding the POI using standard techniques.

In preferred embodiments, the polynucleotides of this disclosure (of a polynucleotide library, for instance) encode at least two or more versions of the POI that each comprise a particular "non-silent" mutation that alters at least one amino acid residue of the POI relative to a reference (*e.g*., wild-type, parental sequence from which the POI encoded by the polynucleotide was derived or is based upon) or other POI, to provide an ROI. The addition, in the form of a synonymous barcode (SynBC) (a first barcode), of at least one silent mutation(s) (which does not result in an amino acid residue change) can be useful in many practical applications such as, for example, to establish experimental replicates with presumably identical phenotype at the stage of polynucleotide synthesis and use the unique first and second barcode mapping provided by the polynucleotide constructs and methods of this disclosure to follow each individually in downstream data analysis, or to make several such versions of the POI, that would otherwise be very similar at the nucleotide level and thus difficult to resolve except by highly accurate DNA sequencing, more diverse at the nucleotide level, and thus easily resolvable even with less-accurate DNA sequencing. Such at least one non-silent mutation(s) can be, and are preferably, made at a region of interest (ROI) of the POI (see, *e.g.,* Fig. 3). Such a mutation can be, for example and without limitation, one or more amino acid substitution(s), deletion(s), addition(s), and/or the like. The one or more mutation(s) can be introduced into the parental polynucleotide(s) by any technique available to those of ordinary skill in the art, including but not limited to DNA synthesis or DNA oligonucleotide ("oligo") synthesis. Such techniques, particularly DNA oligo synthesis, can be used to prepare a library of polynucleotides encoding POIs that differ by one or more amino acids, including but not limited to "site saturation mutagenesis" (SSM), alanine scanning mutagenesis, fully defined mutagenesis, and/or random mutagenesis. The at least one silent mutation included in the first barcode does not encode an amino acid change to the protein-coding region relative to a wild-type, parental, or other version of the protein-coding region (each of which may be a "reference" sequence).

In preferred embodiments, the at least one second barcode (or second (randomized) barcode) is positioned outside, and preferably upstream (5'), of the protein-coding region of the polynucleotides and may be any number of nucleotides and of any polynucleotide sequence. For instance, the second (randomized) barcode can be about any of 5, 10, 15, 20, 25, 30, 35, 40, 45, 50, 55, 60, 65, 70, 75, 80, 85, 90, 95, 100, or more nucleotides, in length, in some preferred embodiments about 25 nucleotides in length (referred to herein as "N25"). In preferred embodiments, each second (randomized) barcode of each member of a polynucleotide preferably has a sufficiently different nucleotide sequence from any other second (randomized) barcode to avoid overlap of the sequences of the second (randomized) barcodes between members of the polynucleotides. Thus, the second (randomized) barcode(s) can comprise any sequence of nucleotides and are preferably sufficiently unique from one another to distinguish one from the other such that the polynucleotide of interest can be identified by sequencing the second (randomized) barcode(s) once pairings have been established between the second (randomized) barcode(s) and the first barcode (SynBC) by either long-read or short-read sequencing. Thus, a polynucleotide library can encode tens, hundreds, thousands, millions, tens of millions, hundreds of millions, or billions of POIs, each being associated with different first (SynBC) and/or second (randomized) barcodes.

The polynucleotides of this disclosure include both a first barcode (SynBC) and a second (randomized) barcode (see, *e.g.,* Fig. 3). The first barcode (SynBC) can be positioned at any position within the protein coding region of the polynucleotide. For instance, the first barcode can be positioned at the 5' (*e.g.,* N-terminal) or the 3' (*e.g*., C-terminal) end of the protein coding region of the polynucleotide. The second (randomized) barcode can be positioned at any position of the polynucleotide outside of the protein coding region of the polynucleotide. The barcodes can optionally be positioned within about 600 nucleotides or less from one another within the polynucleotide. As discussed below, positioning the first and second barcodes within about 600 nucleotides or less from one another within the polynucleotide provides for the use of short-read polynucleotide sequencing to identify one or more polynucleotide(s) that include at least one particular protein coding region (*e.g*., encoding a particular protein). Thus, in some embodiments, this disclosure provides polynucleotides including a protein-coding region, at least one first barcode (*e.g*., SynBC) within the protein coding region, and at least one second (randomized) barcode (*e.g*., N25) positioned outside the protein coding region (see, *e.g.,* Fig. 3), preferably upstream (5') thereof, wherein the polynucleotide(s) including a particular protein-coding region can be identified by the sequence of at least one of the barcodes, and/or in some preferred embodiments both of the barcodes. In some preferred embodiments, following the association (or pairing) of a particular polynucleotide including a particular protein-coding region with first (SynBC) and second (randomized) barcodes (*i.e.,* a second polynucleotide of the second polynucleotide library), the polynucleotide can be identified by the sequence of the second (randomized) barcode alone.

An illustration of a polynucleotide construct encoding a POI and a second barcode is provided by Fig. 1. As shown therein, the second (randomized) barcode (labeled "N25") is positioned outside the coding sequence of the POI (labeled "POI" in Fig. 1). If this second (randomized) barcode were to be positioned inside the coding sequence of the POI, it would corrupt (*e.g*., change the amino acid sequence of, detrimentally affect expression, and/or protein function) the POI. A region of interest ("ROI") within the POI can, in some embodiments, preferably include the first barcode and can additionally include mutations that can affect the amino acid sequence of the POI (non-silent mutations). Fig. 2 illustrates a library of POIs, each including a different ROI, and at least one different second (randomized) barcode (each being indicated as N25 therein). The ROI may consist of the entire POI or may be smaller than the POI (*e.g.,* any of about 10, 20, 30, 40, 50, 60, 70, 80, or 90% as many amino acid residues). The nucleotides encoding the first barcode (SynBC) may optionally be positioned within about 600 nucleotides of the second (randomized) barcode. This is not necessary, however, such as following long-read sequencing of the polynucleotides, since in such embodiments the second (randomized) barcode will have been previously associated with a particular POI and/or ROI and can be the only barcode that requires sequencing to identify or detect that particular polynucleotide. Fig. 3 illustrates an exemplary polynucleotide of a preferred embodiment of this disclosure encoding a POI that includes an ROI in one position of the POI and a first barcode at a different position of the POI (labeled "SynBC"), both therefore within the POI, and a second (randomized) barcode outside the POI coding region (labeled "N25"). In some embodiments, such in the example illustrated in Fig. 3, an intervening section of nucleotides can be present between the second barcode (*e.g*., N25) and the nucleotides encoding the POI. Such an intervening sequence can be any suitable sequence such as, *e.g.,* but not limited to a promoter or a silent linking sequence that does not encode a protein or otherwise function detrimentally affect expression of the POI or sequencing of the first and second barcode, or other suitable sequence. Such constructs may provide for shorter sequence reads being necessary to identify a polynucleotide encoding a particular POI and/or ROI. For instance, in preferred embodiments only the first and second barcodes (*e.g*., N25 and SynBC) need to be sequences, as illustrated in Fig. 3. Polynucleotides of this disclosure, such as the type illustrated in Fig. 3, vastly increases sequence distance between variants, allowing for much better processing of low-quality sequence data. For instance, POI and/or ROI variants may differ at an average of ten nucleotides in barcode constructs (*e.g*., by virtue of the first barcode (SynBC) as illustrated in Fig. 3) as compared to an average of one site within the ROI itself (*e.g*., where the required and/or only variation is found within the ROI).

In some preferred embodiments, this disclosure provides methods for preparing and/or using the first (SynBC) and/or second (randomized) barcodes to identify members of a polynucleotide library comprising a protein-coding region encoding a particular protein-of-interest ("POI"). In preferred embodiments, this disclosure provides methods comprising the steps of: a) synthesizing a first polynucleotide library comprising multiple polynucleotides comprising at least one protein-coding region, each protein-coding region further comprising at least one silent mutation providing the first barcode; b) randomly pairing each polynucleotide of the first polynucleotide library to at least one second (randomized) barcode to produce a second polynucleotide library; c) sequencing the polynucleotides of the second polynucleotide library by long-read nucleotide sequencing (*e.g*., long-read next generation sequencing); and, d) mapping and/or associating each second (randomized) barcode to a protein-coding region; wherein a polynucleotide of the second polynucleotide library can be identified by sequencing only the randomized barcode. The polynucleotide of the second polynucleotide library can be identified by sequencing only the randomized barcode because it was identified as being contained within the polynucleotide comprising a particular protein-coding region in steps c) and d) (*i.e.,* sequencing the polynucleotides of the second polynucleotide library by long-read next generation sequencing; and mapping each second (randomized) barcode to a protein-coding region), which can be performed essentially simultaneously or not, and/or using the same or different systems (*e.g*., physical and/or software systems).

In preferred embodiments, the methods of this disclosure provides methods that comprise (or consist of) identifying a polynucleotide encoding a particular protein-coding sequence by sequencing at least one of the barcodes, preferably the second (randomized) barcode alone, thereof. In some preferred embodiments, the methods of this disclosure comprise (or consist of) sequencing the first barcode and the second (randomized) barcode. In some preferred embodiments, the first barcode and/or second (randomized) barcode(s) are sequenced by short-read sequencing, and even more preferably short-read NGS. In some preferred embodiments, the first barcode and the second (randomized) barcode are separated by less than about 600 nucleotides on the polynucleotides of the polynucleotide library. In some embodiments, the sequencing of step c) comprises partially sequencing the protein-coding region (*e.g*., where the first barcode is sequenced). In some embodiments, the sequencing of step c) comprises fully sequencing the protein-coding region (*e.g*., using long-read sequencing).

In some embodiments, this disclosure provides a polynucleotide library, or polynucleotide libraries, comprising polynucleotides comprising at least one protein-coding region comprising at least one mutation and a first barcode (*e.g*., SynBC); and a second (randomized) barcode (*e.g*., N25) positioned outside the protein-coding region. In preferred embodiments, the first (SynBC) barcode and the second (randomized) barcode are positioned within about 600 or less nucleotides from one another on the polynucleotides. In preferred embodiments, positioning the barcodes within about 600 or less nucleotides from one another in the polynucleotides provides for the ability to use short-read sequencing to identify polynucleotides encoding a particular POI by sequencing the barcodes.

In some embodiments, this disclosure also provides methods comprising determining the identity of a particular POI from a cell comprising a polynucleotide by identifying within the cell or from a cell lysate a polynucleotide comprising a particular second (randomized) barcode prepared as disclosed herein using standard polynucleotide identification methods available to those of skill in the art. In some embodiments, the cell comprising the polynucleotides of this disclosure can be diploid and can be identified by identifying within the cell or from a cell lysate at least two second (randomized) barcodes, each of which was derived from one of a first and a second haploid cell that formed the diploid cell. In some embodiments, identification of the two second (randomized) barcodes indicate the protein encoded by protein-coding region comprised in the first haploid cell interacts with the protein encoded by the protein-coding region of the second haploid cell. In some embodiments, the two second (randomized) barcodes can be associated (*e.g.*, linked, connected, and/or joined) by recombination of loxP sites on the polynucleotides of the polynucleotide library and expression of CRE recombinase in the first and second haploid cells. In some embodiments, the first and second haploid cells are yeast cells, optionally Mat a and Mat α cells, respectively. In some embodiments, a polynucleotide library of first protein binding partners can be assayed against a library of second protein binding partners to identify proteins of interest having binding affinity. The assay used in such embodiments may be the yeast two-hybrid system, the AlphaSeq system (or Yeast Synthetic Agglutination system), or another parallelized high-throughput library-by-library screening method. The AlphaSeq method is described in U.S. Pat. No. 10,988,759 B2, the entire contents of which are hereby incorporated herein into this disclosure for all purposes.

In some embodiments, long-read sequencing platforms may be used to sequence a portion of a construct including a synthetic user-designated barcode sequence outside of a nucleotide sequence encoding a protein of interest (POI) (*e.g.,* the second (randomized) barcode), the nucleotide sequence encoding the protein of interest (POI) (that in preferred embodiments includes the first barcode/SynBC (see, *e.g.,* Fig. 3)), and a nucleotide sequence encoding region of interest (ROI) with the protein of interest (POI) (*e.g.,* the mutation(s) of interest). Long-read sequencing platforms that can be used may include, but are not limited to, for example, Oxford Nanopore Technologies' (ONT) MinION, GridION, or PromethION platforms, or Pacific Biosciences' Sequel System single-molecule real-time (SMRT) platform. While the accuracy of ONT and SMRT reads has increased in recent versions of the respective platforms, both ONT and SMRT technologies provide lower per read accuracy that short-read sequencing, *e*.*g*., such as but not limited to Illumina sequencing platforms (Amarasinghe, S.L., Su, S., Dong, X. et al. Opportunities and challenges in long-read sequencing data analysis. Genome Biol 21, 30 (2020)). While a per-read accuracy of ~95% may be acceptable for mapping a read to a reference sequence, that level of accuracy is often not sufficient to assign a read of a nucleotide sequence encoding a protein of interest (POI), which may differ from other members of a library of mutant / mutated polynucleotides (or mutants) (*e.g*. members of a site-saturation mutagenesis library) by only a single base-pair (or nucleotide), to a unique user-designated synthetic barcode positioned outside the nucleotide sequence encoding the protein of interest (POI) (*e.g.,* the second barcode labeled N25 as shown in Fig. 3). If the region of interest (ROI) within the nucleotide sequence encoding the POI is in excess of ~ 1 kilobase (kb) away from the beginning (or first nucleotide) of the ORF, long-read sequencing platforms may be required to sequence from the user-designated synthetic nucleotide barcode (*i.e.,* the second randomized barcode) position outside of the nucleotide sequence encoding the protein of interest (POI), through the nucleotide sequence encoding the protein of interest, and further through the nucleotide sequence encoding the region of interest (ROI). In some embodiments, the first barcode (SynBC) may comprise one, two, three, four, five, six, seven, eight, nine, ten, or more "synonymous" (meaning the amino acid sequence encoded thereby is not changed relative to a reference sequence) single-nucleotide substitutions within one, two, three, four, five, six, seven, eight, nine, ten, or more codons in the open reading frame (ORF) of a polynucleotide encoding a POI. As mentioned above, as a result of introducing the first barcode (SynBC) into the polynucleotide, the amino acid sequence of the protein of interest remains unchanged while the DNA sequence distance (*i.e.,* the extent of sequence diversity) between the nucleotide sequence encoding any two variant proteins of interest is massively increased relative to the modest DNA sequence distance present without the SynBC. This increased sequence diversity between the nucleotide sequence encoding any two variant proteins of interest within a library allows for enhanced resolvability using error-prone long-read sequencing platforms, preferably when used in conjunction with the second (randomized) barcode.

In an embodiment, the synonymous barcode (SynBC) and the region of interest (ROI) may be synthesized as a single DNA molecule, *i.e.,* as a single synthetic oligonucleotide. The synonymous barcode (SynBC) and the region of interest (ROI) may be user-designed and synthesized by any number of commercial oligonucleotide synthesis methods that are well known in the art and commercially available.

In some embodiments, this disclosure provides methods for identifying a cell (haploid or diploid) comprising a first polynucleotide encoding a protein of interest comprising a modified amino acid sequence, the method comprising sequencing a second polynucleotide comprising a first barcode positioned within the first polynucleotide and a second (randomized) barcode positioned outside the first polynucleotide. In some embodiments, the second polynucleotide could include at least one combination of barcodes that each originated in the haploid cells (*e.g.,* each haploid cell having included a first barcode (SynBC) and a second (randomized) barcode).

In some embodiments, this disclosure provides methods for identifying interacting proteins, the method comprising co-culturing first and second haploid yeast cells (*e.g.,* Mat a, Mat α) that each comprise at least one exogenous coding sequence encoding first or second POIs, respectively, and at least one first barcode within each exogenous coding sequence, each cell further comprising at least one second (randomized) barcode outside the exogenous coding sequence, wherein mating of the first and second haploid yeast cell indicates the first and second proteins interact, the method further comprising identifying the first and second proteins by sequencing the barcodes. In some such embodiments, the barcodes derived from each of the haploid cells are recombined into a single polynucleotide that is sequenced to identify the first and second proteins. In some such embodiments, this disclosure provides methods comprising: a) co-culturing a first haploid yeast strain (*e.g*., Mat a) and a second haploid yeast strain *(e.g.,* Mat α), wherein: the first haploid yeast strain comprises a first polynucleotide comprising at least first and second synthetic polynucleotide barcodes (SynBCs), a first lox P site, and a first coding sequence encoding a first protein of interest (POI) expressed on the cell surface (*e.g*., an antigen (Ag)); the second haploid yeast strain comprises a second polynucleotide comprising at third and fourth SynBCs, a second lox P site, and a second coding sequence encoding a second POI expressed on the cell surface (*e.g*., an antibody (ab)); the first and third SynBCs are positioned outside the first and second coding sequences, respectively (*e.g.,* N25); the second and fourth SynBCs are positioned within the first and second coding sequences, respectively (*e.g.,* Ag in Mat a and Ab in Mat α) one of the first or second haploid yeast strains comprises an expression cassette comprising an inducible promoter operably linked to a CRE recombinase, the other of the first or second haploid yeast strains constitutively expresses a ligand capable of inducing the inducible promoter to induce expression of CRE; the first and second haploid yeast strains comprise complementary selectable markers; the first and second proteins being potential binding partners; b) selecting for diploid cells; c) inducing the expression of CRE recombinase in the diploid cells resulting in recombination through the first and second loxP sites to produce target polynucleotides comprising first and third SynBCs or second and fourth SynBCs; d) sequencing the first and third SynBCs and second and fourth SynBCs; wherein sequencing of the first and third SynBCs and second and fourth SynBCs of the diploid cell indicates the first and second proteins are binding partners.

In some embodiments, this disclosure provides cells (*e.g.,* a haploid yeast cell) comprising at least one exogenous coding sequence encoding at least one POI, at least one first barcode (SynBC) positioned within the exogenous coding sequence, and at least one second synthetic barcode positioned outside the exogenous coding sequence.

In some embodiments, this disclosure provides a combination of polynucleotides disclosed herein, the combination comprising multiple species of polynucleotides, each species comprising a different randomized barcode (*e.g.,* N25 (see, *e.g.,* Fig. 3)) and at least one different first barcode (SynBC) within the polynucleotide encoding the POI. In some such embodiments, the polynucleotides comprising at least one open reading frame (ORF) encoding a POI comprising at least one ROI comprising at least one mutation; at least one randomized barcode upstream (5') or downstream (3') of the ORF; at least one first polynucleotide barcode (SynBC) within the ORF and being positioned upstream (5') or downstream (3') of the at least one randomized barcode. In preferred embodiments, the at least one first polynucleotide barcode (SynBC) and the second (randomized) barcode are within about 600 nucleotides of one another on the polynucleotide. In preferred embodiments, the second (randomized) barcode is randomly generated. In some embodiments, at least some, or all, of the polynucleotides include a codon variation in the nucleotide sequence encoding the wild-type or parental protein encoded by the ORF, wherein the codon variation does not change the amino acid sequence of the POI. In other embodiments, at least some, or all, of the polynucleotides include a codon variation in the nucleotide sequence encoding the wild-type or parental protein encoded by the ORF, wherein the codon variation does change the amino acid sequence of the POI. In some embodiments, the first barcode (SynBC) is positioned between the randomized barcode and polynucleotides encoding the ROI. In some embodiments, the first barcode (SynBC) is positioned within the polynucleotides encoding the ROI. In some embodiments, the ORF includes a 5' end and a 3' end and the ROI, the first barcode, and/or the second (randomized) barcode is positioned closer to the 5' end than to the 3' end of the polynucleotide. In some embodiments, this disclosure provides a combination of multiple species of polynucleotides, each species comprising a different 25-nucleotide second barcode (or other appropriate length second barcode) and at least one different first barcode (SynBC). In some embodiments, such a combination can include multiple species mutational variants of the ROI. In some embodiments, this disclosure provides cells, or a combination of cells, comprising one or more of such polynucleotides and/or combinations thereof.

In some embodiments, such cells are yeast cells. In some embodiments, such cells can comprise a cell surface and display the POI thereupon. In some embodiments, once the barcode has been mapped and associated the random barcode with the protein coding variant (*e.g*., a POI with a particular mutation), the user will be able to: conduct an AlphaSeq assay using short-read NGS to identify proteins that have bound to one another (*e.g*., as described in the above-mentioned U.S. Pat. No. 10,988,759 B2); identify enriched antibodies from a phage pan using short read NGS; and/or, conduct other protein engineering / molecular biology assays (cell-free or using cells (*e.g*., mammalian, yeast, bacterial, insect, or other type of cell)) that would benefit from using short read sequencing to identify protein variants from a library that was at least partially synthesized. In some embodiments, the POI may be short (*e.g.,* a peptide of less than 50 amino acid residues) and the entire POI is synthesized as a SynBC. And in some embodiments, such as where a POI is long (*e.g.,* more than 1000 amino acid residues), an ROI can be synthesized and cloned into a polynucleotide encoding the remainder of the POI. Other embodiments are also contemplated by this disclosure as will be understood by those of ordinary skill in the art.

This disclosure provides, in some embodiments, methods comprising synthesizing a first polynucleotide library comprising multiple polynucleotides comprising at least one protein-coding region (or region of interest within a protein coding region) comprising at least one mutation, each protein-coding region further comprising at least one silent mutation providing a first barcode; randomly pairing each polynucleotide of the first polynucleotide library to at least one second (randomized) barcode to produce a second polynucleotide library; sequencing the polynucleotides of the second polynucleotide library, optionally by long-read next generation sequencing; and, mapping each second (randomized) barcode to a protein-coding region; wherein a polynucleotide of the second polynucleotide library can be identified by sequencing only the randomized barcode. In some embodiments, the methods comprise sequencing the second (randomized) barcodes. In some embodiments, the methods comprise identifying a polynucleotide encoding a particular protein-coding sequence by sequencing the second (randomized) barcode thereof. In some embodiments, the methods comprise sequencing the first barcode and the second (randomized) barcode. In some embodiments, the methods comprise sequencing the first barcode and/or second (randomized) barcode(s) by short-read next-generation sequencing. In some embodiments, the first barcode and the second (randomized) barcode are separated by more than about 300 nucleotides on the polynucleotides.

In some embodiments, the methods comprise partially sequencing the protein-coding region. In some embodiments, the methods comprise fully sequencing the protein-coding region. In some embodiments, the first barcode comprises one to 100 nucleotide differences from a wild-type, parental or reference sequence of the protein-coding region. In some embodiments, all or most, preferably all, mutations in the first barcode are phenotypically silent. In some embodiments, the mutation(s) is not phenotypically silent. In some embodiments, the methods comprise isolating a cell comprising a polynucleotide encoding a particular protein-coding region by identifying within the cell a particular second (randomized) barcode. In some embodiments, the cell is diploid and is identified by identifying within the cell at least two second (randomized) barcodes, each of which was derived from one of a first and a second haploid cell that formed the diploid cell.

In some embodiments, the methods comprise identification of the two second (randomized) barcodes to indicate the protein encoded by protein-coding region comprised in the first haploid cell interacts with the protein encoded by the protein-coding region of the second haploid cell. In some embodiments, the methods comprise the two second (randomized) barcodes are associated by recombination of loxP sites on the polynucleotides of the polynucleotide library and expression of CRE recombinase in the first and second haploid cells. In some embodiments, the first and second haploid cells are yeast cells, optionally Mat a and Mat α cells, respectively.

In some embodiments, this disclosure provides and/or comprises one or more polynucleotide libraries comprising polynucleotides comprising at least one protein-coding region comprising at least one mutation and a first barcode; and a second (randomized) barcode positioned outside the protein-coding region. In some embodiments, the polynucleotides of the polynucleotide library include a first barcode and the second (randomized) barcode and are positioned within about 600 or less nucleotides from one another on the polynucleotides. In some embodiments, the polynucleotides of the library comprise at least one protein-coding region comprising at least one mutation and a first barcode; and a second (randomized) barcode positioned outside the protein-coding region. In some embodiments, the first barcode and the second (randomized) barcode are positioned within about 600 or less nucleotides from one another.

Other aspects (or embodiments) are also contemplated by this disclosure as will be understood by those of ordinary skill in the art.

Thus, in preferred embodiments, this disclosure provides methods comprising: a) synthesizing a first polynucleotide library comprising multiple first polynucleotides each comprising at least one protein-coding region, and/or encoding at least one region of interest within a protein-coding region, each first polynucleotide independently and optionally comprising one or more non-silent mutations with respect to a reference sequence of the protein-coding region and/or region of interest, at least one of the first polynucleotides encoding the protein-coding region or region of interest comprising at least one silent mutation with respect to the reference sequence, the at least one silent mutation or a combination of silent mutations in a given protein-coding region and/or region of interest providing a first barcode; b) randomly pairing at least one first polynucleotide to a unique second randomized barcode nucleotide sequence to produce a second polynucleotide library comprised of second polynucleotides; c) sequencing the second polynucleotides or at least the first barcode and the second randomized barcode thereof; and, d) mapping each second randomized barcode to a protein-coding region and/or region of interest of a first polynucleotide; wherein a polynucleotide of the second polynucleotide library can be identified by sequencing only the randomized barcode. "Randomly pairing" of each first polynucleotide to at least one (most preferably one) second randomized barcode nucleotide sequence to produce a second polynucleotide library comprised of second polynucleotides can be accomplished by any method known in the art for joining polynucleotides to one another including but not limited to ligation, linking, combining, joining, and/or producing joined polynucleotides using standard ligation techniques, polymerase chain reaction (PCR), and the like. In preferred embodiments, "randomly pairing" means the method does not require any particular first polynucleotide to be paired with any particular second randomized barcode. The term "unique second randomized barcode nucleotide sequence" means that each first polynucleotide encoding a particular POI (including a particular ROI and SynBC) is paired to a second randomized barcode nucleotide sequence that is unique relative to any other first polynucleotide encoding that particular POI such that each first polynucleotide, or cell comprising the same, can be identified by sequencing the second randomized barcode nucleotide sequence (with or without the SynBC). In some such preferred embodiments, the second polynucleotides can be sequenced by long-read next generation sequencing. In some such preferred embodiments, the second (*i.e.,* randomized) barcode is sequenced using short-read next generation sequencing. In some such preferred embodiments, the methods can include identifying a polynucleotide encoding a particular protein-coding sequence (preferably polynucleotide encoding a POI) by sequencing the second (randomized) barcode thereof. In some such preferred embodiments, the methods include comprising determining the identities and relative abundances of polynucleotides encoding one or more protein-coding sequences by sequencing the second randomized barcodes thereof. In some such preferred embodiments, the first barcode and/or second (randomized) barcode(s) can be sequenced by short-read next-generation sequencing, such as with an Illumina platform. In some such preferred embodiments, the polynucleotides of the second polynucleotide library contain first barcodes and second (randomized) barcodes that are separated by more than about 300 nucleotides. In some such preferred embodiments, both the first barcode and the second (randomized) barcode contained within the polynucleotides of the second polynucleotide library are sequenced by long-read next-generation sequencing, such as nanopore or PacBio sequencing. In some such preferred embodiments, the polynucleotides of the second polynucleotide library contain first barcodes and second (randomized) barcodes that are separated by less than about 600 nucleotides. In some such preferred embodiments, both the first barcode and the second (randomized) barcode contained within the polynucleotides of the second polynucleotide library are sequenced by short-read next-generation sequencing, such as Illumina sequencing. In some such preferred embodiments, the first polynucleotide library contains one or more polynucleotides that contain a protein-coding region coding for a protein with a single amino acid mutation with respect to a reference protein sequence. In some such preferred embodiments, one or more polynucleotides from the first polynucleotide library contains a single non silent mutation resulting from a single nucleic acid substitution. In some such preferred embodiments, the first barcode comprises three or more silent mutations. In some such preferred embodiments, one or more polynucleotides from a first polynucleotide library contains more nucleic acid mutations with respect to a reference protein sequence that encode to silent mutations compared to the number of nucleic acid mutations with respect to a reference protein sequence that encode non-silent mutations. In some such preferred embodiments, two or more polynucleotides from a second polynucleotide library contain identical non-silent mutations with respect to a reference protein sequence but different second barcodes such that the two molecules encoding an identical amino acid sequence (*i.e.,* acting as biological replicates) are identified by sequencing the second barcodes. In some such preferred embodiments, one or more protein coding regions in one or more cells are identified by sequencing one or more second barcodes. In some such preferred embodiments, two protein coding regions in one or more cells are identified by sequencing two second barcodes contained within the same cell. In some such preferred embodiments, the cell is a yeast diploid cell. In some such preferred embodiments, the yeast diploid cell is produced through the mating of two yeast haploid cells, each comprising one second barcode. Other embodiments are also contemplated by this disclosure, as will be understood by those of ordinary skill in the art.

Unless defined otherwise, all technical and scientific terms used herein have the same meaning as commonly understood by one of ordinary skill in the art to which this invention belongs. All publications mentioned herein are for the purpose of describing and disclosing devices, methods and cell populations that may be used in connection with the presently described invention.

Reference throughout the specification to "one embodiment" or "an embodiment" means that a particular feature, structure, or characteristic described in connection with an embodiment is included in at least one embodiment of the subject matter disclosed. Thus, the appearance of the phrases "in one embodiment" or "in an embodiment" in various places throughout the specification is not necessarily referring to the same embodiment. Further, the particular features, structures or characteristics may be combined in any suitable manner in one or more embodiments. Further, it is intended that embodiments of the disclosed subject matter cover modifications and variations thereof.

It must be noted that, as used in the specification and the appended claims, the singular forms "a," "an," and "the" include plural referents unless the context expressly dictates otherwise. That is, unless expressly specified otherwise, as used herein the words "a," "an," "the," and the like carry the meaning of "one or more." Additionally, it is to be understood that terms such as "left," "right," "top," "bottom," "front," "rear," "side," "height," "length," "width," "upper," "lower," "interior," "exterior," "inner," "outer," and the like that may be used herein merely describe points of reference and do not necessarily limit embodiments of the present disclosure to any particular orientation or configuration. Furthermore, terms such as "first," "second," "third," etc., merely identify one of a number of portions, components, steps, operations, functions, and/or points of reference as disclosed herein, and likewise do not necessarily limit embodiments of the present disclosure to any particular configuration or orientation. Furthermore, the terms "approximately," "about," "proximate," "minor variation," and similar terms generally refer to ranges that include the identified value within a margin of 20%, 10% or preferably 5% in certain embodiments, and any values therebetween.

All of the functionalities described in connection with one embodiment are intended to be applicable to the additional embodiments described below except where expressly stated or where the feature or function is incompatible with the additional embodiments. For example, where a given feature or function is expressly described in connection with one embodiment but not expressly mentioned in connection with an alternative embodiment, it should be understood that the inventors intend that that feature or function may be deployed, utilized or implemented in connection with the alternative embodiment unless the feature or function is incompatible with the alternative embodiment.

The practice of the techniques described herein may employ, unless otherwise indicated, conventional techniques and descriptions of organic chemistry, polymer technology, molecular biology (including recombinant techniques), cell biology, cell culture, biochemistry, and sequencing technology, which are within the skill of those who practice in the art. Such conventional techniques include bacterial, fungal, and mammalian cell culture techniques and screening assays. Specific illustrations of suitable techniques can be had by reference to the examples herein. However, other equivalent conventional procedures can, of course, also be used. Such conventional techniques and descriptions can be found in standard laboratory manuals such as Green, et al., Eds. (1999), Genome Analysis: A Laboratory Manual Series (Vols. I-IV); Weiner, Gabriel, Stephens, Eds. (2007), Genetic Variation: A Laboratory Manual; Dieffenbach, Dveksler, Eds. (2003), PCR Primer: A Laboratory Manual; Bowtell and Sambrook (2003), DNA Microarrays: A Molecular Cloning Manual; Mount (2004), Bioinformatics: Sequence and Genome Analysis*;* Sambrook and Russell (2006), Condensed Protocols from Molecular Cloning: A Laboratory Manual*;* and Sambrook and Russell (2002), Molecular Cloning: A Laboratory Manual (all from Cold Spring Harbor Laboratory Press); Stryer, L. (1995) Biochemistry (4th Ed.) W.H. Freeman, New York N.Y.; Gait, "Oligonucleotide Synthesis: A Practical Approach" 1984, IRL Press, London; Nelson and Cox (2000), Lehninger, Principles of Biochemistry 3rd Ed., W. H. Freeman Pub., New York, N.Y.; Berg et al. (2002) Biochemistry, 5th Ed., W.H. Freeman Pub., New York, N.Y.; all of which are herein incorporated in their entirety by reference for all purposes.

The term "complementary" as used herein refers to Watson-Crick base pairing between nucleotides and specifically refers to nucleotides hydrogen bonded to one another with thymine or uracil residues linked to adenine residues by two hydrogen bonds and cytosine and guanine residues linked by three hydrogen bonds. In general, a nucleic acid includes a nucleotide sequence described as having a "percent complementarity" or "percent homology" to a specified second nucleotide sequence. For example, a nucleotide sequence may have 80%, 90%, or 100% complementarity to a specified second nucleotide sequence, indicating that 8 of 10, 9 of 10 or 10 of 10 nucleotides of a sequence are complementary to the specified second nucleotide sequence. For instance, the nucleotide sequence 3'-TCGA-5' is 100% complementary to the nucleotide sequence 5'-AGCT-3'; and the nucleotide sequence 3'-TCGA-5' is 100% complementary to a region of the nucleotide sequence 5'-TTAGCTGG-3'. "Homology" or "identity" or "similarity" refers to sequence similarity between two peptides or, more often in the context of the present disclosure, between two nucleic acid molecules. The term "homologous region" or "homology arm" refers to a region on the donor DNA with a certain degree of homology with the target genomic DNA sequence. Homology can be determined by comparing a position in each sequence which may be aligned for purposes of comparison. When a position in the compared sequence is occupied by the same base or amino acid, then the molecules are homologous at that position. A degree of homology between sequences is a function of the number of matching or homologous positions shared by the sequences. As used herein, the term "vector" is any of a variety of nucleic acids that comprise a desired sequence or sequences to be delivered to and/or expressed in a cell. Vectors are typically composed of DNA, although RNA vectors are also available. Vectors include, but are not limited to, plasmids, fosmids, phagemids, virus genomes, BACs, YACs, PACs, synthetic chromosomes, among others. "Operably linked" refers to an arrangement of elements, *e*.*g*., barcode sequences, gene expression cassettes, coding sequences, promoters, enhancers, transcription factor binding sites, where the components so described are configured so as to perform their usual function. Thus, control sequences operably linked to a coding sequence are capable of effecting or controlling the transcription, and in some cases, the translation, of a coding sequence. The control sequences need not be contiguous with the coding sequence so long as they function to direct the expression of the coding sequence. Thus, for example, intervening untranslated yet transcribed sequences can be present between a promoter sequence and the coding sequence and the promoter sequence can still be considered "operably linked" to the coding sequence. In fact, such sequences need not reside on the same contiguous DNA molecule (*e.g*., chromosome) and may still have interactions resulting in altered regulation.

Amino acids may be referred to by standard single letter abbreviations and encoded by codons as is known in the art, including but not limited to those shown below in **Table 1**:

**Table 1**

| Amino acids | Symbols | | Codons |
|---|---|---|---|
| Alanine | Ala | A | GCA, GCC, GCG, GCU |
| Cysteine | Cys | C | UGC, UGU |
| Aspartic acid | Asp | D | GAC, GAU |
| Glutamic acid | Glu | E | GAA, GAG |
| Phenylalanine | Phe | F | UUC, UUU |
| Glycine | Gly | G | GGA, GGC, GGG, GGU |
| Histidine | His | H | CAC, CAU |
| Isoleucine | Ile | I | AUA, AUC, AUU |
| Lysine | Lys | K | AAA, AAG |
| Leucine | Leu | L | UUA, UUG, CUA, CUC, CUG, CUU |
| Methionine | Met | M | AUG |
| Asparagine | Asn | N | AAC, AAU |
| Proline | Pro | P | CCA, CCC, CCG, CCU |
| Glutamine | Gln | Q | CAA, CAG |
| Arginine | Arg | R | AGA, AGG, CGA, CGC, CGG, CGU |
| Serine | Ser | S | AGC, AGU, UCA, UCC, UCG, UCU |
| Threonine | Thr | T | ACA, ACC, ACG, ACU |
| Valine | Val | V | GUA, GUC, GUG, GUU |
| Tryptophan | Trp | W | UGG |
| Tyrosine | Tyr | Y | UAC UAU |

As used herein the term "selectable marker" refers to a gene introduced into a cell, which confers a trait suitable for artificial selection. General use selectable markers are well-known to those of ordinary skill in the art. Drug selectable markers such as ampicillin/carbenicillin, kanamycin, chloramphenicol, erythromycin, tetracycline, gentamicin, bleomycin, streptomycin, puromycin, hygromycin, blasticidin, and G418 may be employed. A selectable marker may also be an auxotrophy selectable marker, wherein the cell strain to be selected for carries a mutation that renders it unable to synthesize an essential nutrient. Such a strain will only grow if the lacking essential nutrient is supplied in the growth medium. Essential amino acid auxotrophic selection of, for example, yeast mutant strains, is common and well-known in the art. "Selective medium" as used herein refers to cell growth medium to which has been added a chemical compound or biological moiety that selects for or against selectable markers or a medium that is lacking essential nutrients and selects against auxotrophic strains.

As used herein, "affinity" is the strength of the binding interaction between a single biomolecule to its ligand or binding partner. Affinity is usually measured and described using the equilibrium dissociation constant, *K_{D}.* The lower the *K_{D}* value, the greater the affinity between the protein and its binding partner. Affinity may be affected by hydrogen bonding, electrostatic interactions, hydrophobic and Van der Waals forces between the binding partners, or by the presence of other molecules, *e*.*g*., binding agonists or antagonists.

As used herein, "site saturation mutagenesis" (SSM), refers to a mutagenesis technique used in protein engineering and molecular biology, wherein a codon or set of codons is substituted with most or all possible amino acids at the position in the polypeptide. SSM may be performed for one codon, several codons, or for every position in the protein. The result is a library of mutant proteins representing the full or nearly full complement of possible amino acids at one, several, or every amino acid position in a polypeptide. Sometimes particular amino acids are excluded from a site-saturation mutation (SSM) library, such as cysteines, that may have unwanted effects on protein folding or function.

As used herein, "silent mutation" or "synonymous substitution" refers to a substitution of one nucleotide base for another in a coding region of a nucleotide sequence of interest (*e.g*., polynucleotide) such that the amino acid sequence encoded by the nucleotide sequence of interest (*e.g*., encoding the protein of interest (POI)) is not modified.

In some embodiments, synonymous barcodes (first barcode or SynBC) may be introduced to the open reading frame of a protein of interest to introduce sequence diversity among individual members of a mutagenic library and provide for the identification and deconvolution of members of the mutagenic library during downstream processing of sequencing data generated for the library by high-throughput assays. Due to the degenerate nature of the genetic code, one or more single nucleotide substitutions may be introduced to the DNA sequence of an open reading frame encoding a protein of interest without altering the amino acid sequence of the translated polypeptide. For example, the hypothetical DNA sequence "ATG GCC GAA ..." which encodes "Met-Ala-Glu ..." may be changed to "ATG GCA GAA ..." while still encoding "Met-Ala-Glu." The single nucleotide substitution of an adenine for a cytosine at the third position of the second codon is a synonymous, and thus "silent," substitution. In some implementations, silent substitutions may be introduced to one, two, three, four, five, six, seven, eight, nine, ten, or more codons in the ORF encoding a protein of interest. Depending on the original codon, in some implementations one, two, three, four, or five synonymous substitutions may be introduced to the DNA sequences of the ORF, with each unique substitution or combination of substitutions corresponding to a member of the mutagenic library.

Other embodiments are also contemplated herein as would be understood by those of ordinary skill in the art.

### EXAMPLES

The following examples are put forth so as to provide those of ordinary skill in the art with a complete disclosure and description of how to use the embodiments provided herein and are not intended to limit the scope of the disclosure nor are they intended to represent that the Examples below are not necessarily all of the experiments or the only experiments performed. Efforts have been made to ensure accuracy with respect to numbers used (*e.g*., amounts, temperature, etc.) but some experimental errors and deviations should be accounted for. Unless indicated otherwise, parts are parts by volume, and temperature is in degrees Centigrade. It should be understood that variations in the methods as described can be made without changing the fundamental aspects that the Examples are meant to illustrate.

To evaluate the effectiveness of utilizing first barcodes (SynBCs) in the open reading frame ("ORF") of individual SAPs as well as second (second (randomized) barcodes) outside the ORF a library of SAPs was produced without SynBCs and a library of SAPs was produced with SynBCs according to the methods disclosed herein. Each library comprised thousands of mutational variants of proteins of interest (POIs) generated using site saturation mutagenesis (SSM) at regions of interest.

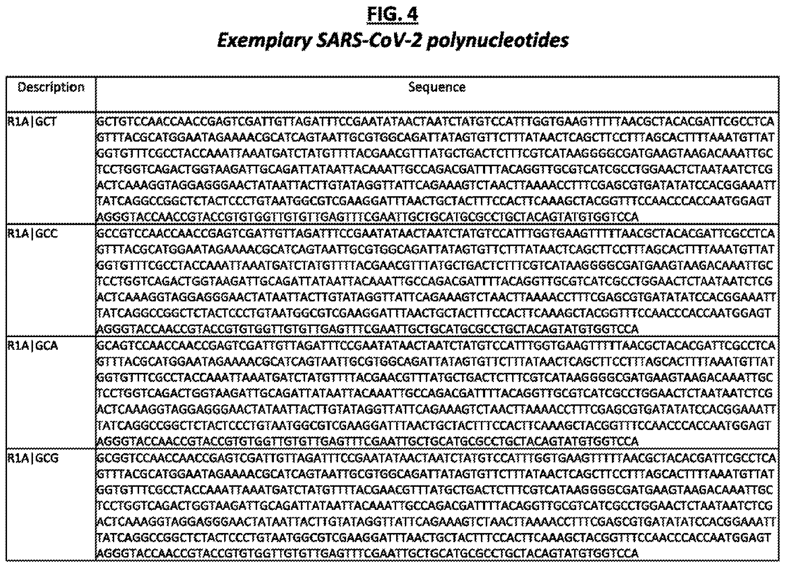

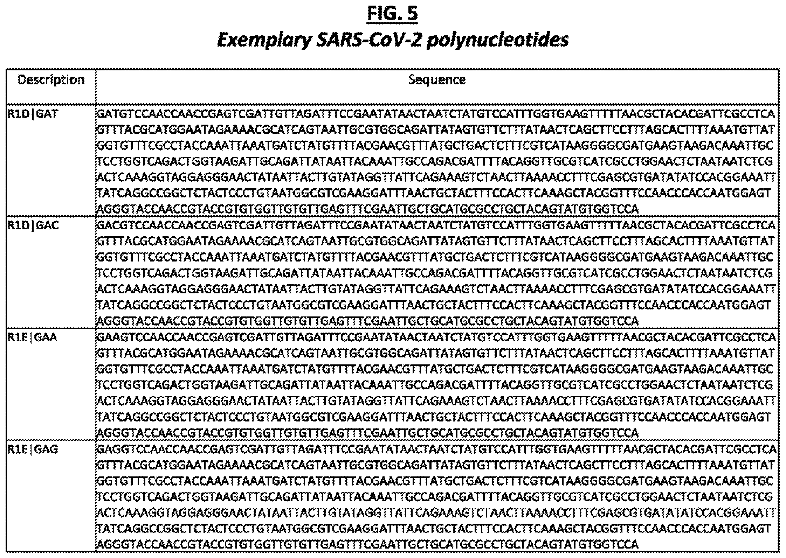

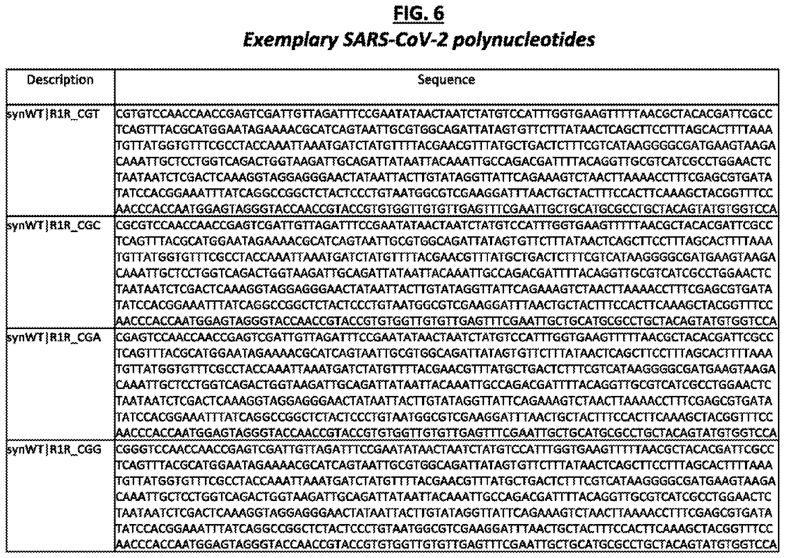

A first exemplary group of polynucleotides encode the SARS-CoV-2 spike protein Receptor Binding Domain (RBD) and mutants thereof (AAYL75 (SARS-CoV2-RBD SSM)). Figs. 4-6 provide the expected DNA sequence of all elements of a Site Saturation Mutagenesis (SSM) library covering exemplary RBD residues. In Figs. 4-6, the "Description" provides the mutant name and the "Sequence" column provides the DNA sequence encoding the mutant RBD. All amino acid changes are indicated using standard single letter amino acid symbols. For instance, R1A codes for SARS-CoV2 RBD with an Arginine (R) to Alanine (A) mutation at the first amino acid position within the mutated region of the RBD polypeptide (*e.g*., R1A).

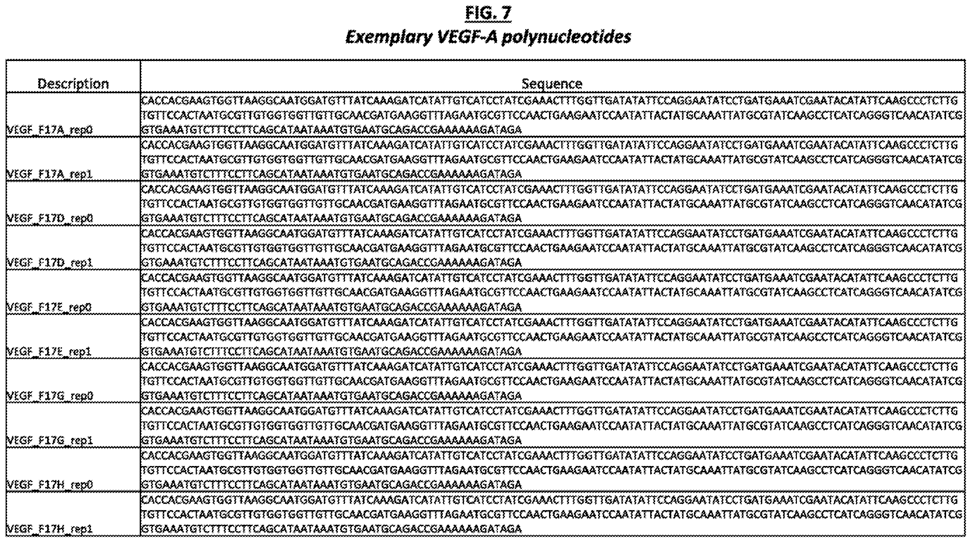

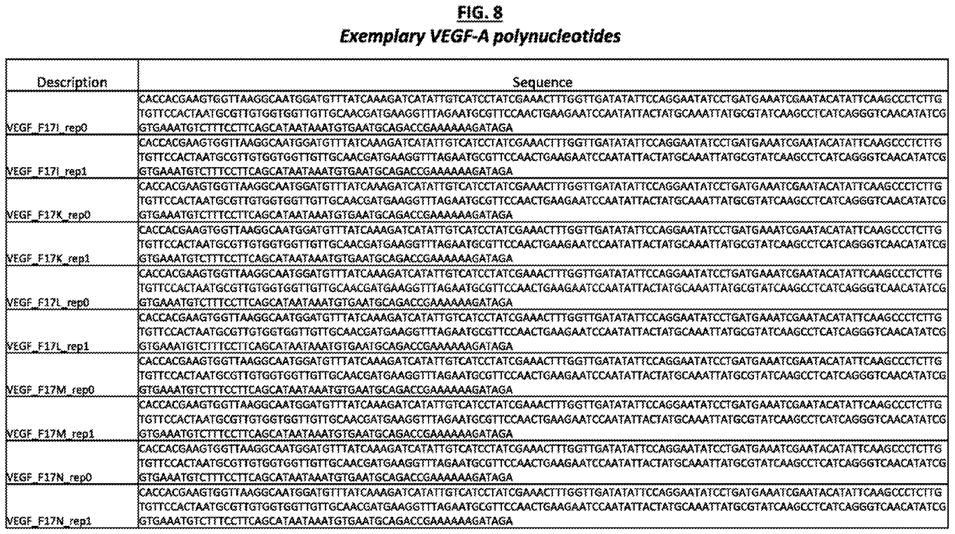

A second exemplary group of polynucleotides encode vascular endothelial growth factor A (VEGF-A) (AAYL135 (VEGF SSM)). Figs. 7-8 provide the expected DNA sequence of all elements of a Site Saturation Mutagenesis (SSM) library of select residues of VEGF-A. In Figs. 7-8, the "Description" column provides gives the mutant name and the "Sequence" column provides the DNA sequence. All amino acid changes are indicated using standard single letter amino acid symbols. For instance, the description VEGF_F17A_rep0 codes for the first replicate (of 2) of VEGF-A with position 17 mutated from Phenylalanine (F) to Alanine (A) (*i.e.,* indicated by "F17A").

In a library comprising mutants with a single amino-acid change such as the VEGF-A and RBD examples shown here, all DNA sequences are quite similar making it difficult to distinguish between the variants using low-quality DNA sequencing data. The first barcode (SynBC) as disclosed herein surprisingly and significantly increases the ability of the user to distinguish such polynucleotides and pair them with a second (randomized) barcode. The SynBC and mutation(s) have been synthesized in a fully-defined manner. As such, identification of the SynBC (which differs from all other SynBCs by many nucleotide differences) allows the user to infer that a particular mutational variant has been identified. The randomized barcodes also differ considerably from each other, so it is simple for the user to determine for a given SynBC, allowing the user to easily make the association between the two barcodes.

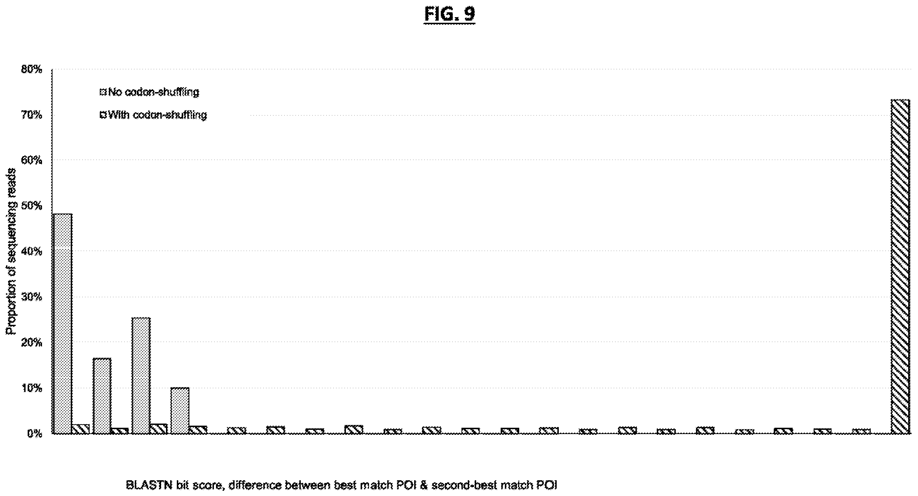

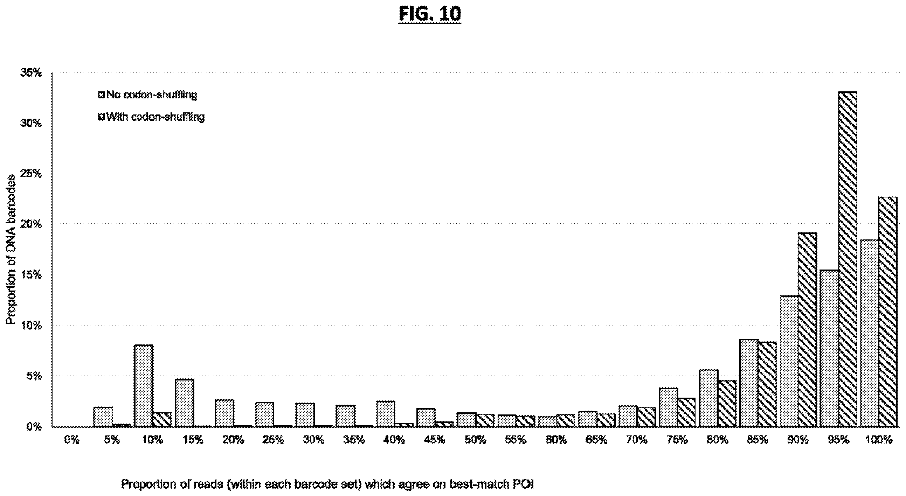

For the library including the first (SynBC) and second (randomized) barcodes, each mutational variant was assigned a known and unique SynBC within the ORF encoding the POI and a second (randomized) barcode outside the ORF encoding the POI. After insert synthesis and library construction, each library (*i.e.,* with and without the first (SynBC) and second (randomized) barcodes) was sequenced using long-read next generation sequencing (MinION platform, Oxford Nanopore Technologies). Sequencing reads of the ORFs encoding the POIs were aligned to expected POI sequencing and alignments were scored using the BLASTN tool (Altschul, S.F., Gish, W., Miller, W., Myers, E.W. & Lipman, D.J. (1990) "Basic local alignment search tool." J. Mol. Biol. 215:403-410). Figs. 9 and 10 show histogram plots of sequencing reads comprising second (randomized) barcodes in addition to POI sequence with and without, respectively (indicated as "No codon shuffling"), the first (SynBC) barcode. Sequence reads are binned by the difference in BLASTN score between the best match to each sequencing read to the set of expected POI sequences and the second-best match. A low BLASTN score difference indicates that it is difficult or impossible to distinguish between sequence reads due to limited sequence divergence between library members and the relatively high rate of sequencing errors of the long-read sequencing platform. A high BLASTN score indicates that sequencing reads of POI constructs are identifiable and that an individual POI may be unambiguously paired to its unique second (randomized) barcodes. With no SynBC barcodes, many POI sequences are nearly identical and due to sequencing errors, nearly 50% of sequencing reads are rendered useless by being equally distant from two or more expected POI sequences, *i.e.,* sequencing reads and therefore particular second (randomized) barcodes cannot be assigned to an individual POI. The raw data is shown below in Tables 2 and 3. With the barcodes, more than 90% of sequencing reads may be unambiguously assigned to a unique POI. Highly certain barcode-POI association (greater than 95% agreement among the various sequencing reads including a particular second (random) barcode) is seen for 34% of random barcodes without SynBC barcodes and for 56% of random barcodes with SynBC barcodes.

**Table 2**

| ***BLAST_margins*** | | | | | | |
|---|---|---|---|---|---|---|
| AAYL75 (SARS-CoV2-RBD SSM) | | | | AAYL135 (VEGF SSM) | | |
| BLAST score margin | N | % | | BLAST score margin | N | % |
| 0 | 155169 | 0.481634 | | 0 | 6597 | 0.019996 |
| 1 | 53078 | 0.164751 | | 1 | 3894 | 0.011803 |
| 2 | 81758 | 0.253771 | | 2 | 6657 | 0.020178 |
| 3 | 32098 | 0.09963 | | 3 | 5288 | 0.016028 |
| 4 | 13 | 4.04E-05 | | 4 | 4388 | 0.0133 |
| 5 | 26 | 8.07E-05 | | 5 | 5041 | 0.015279 |
| 6 | 5 | 1.55E-05 | | 6 | 3376 | 0.010233 |
| 7 | 16 | 4.97E-05 | | 7 | 5684 | 0.017228 |
| 8 | 3 | 9.31E-06 | | 8 | 3105 | 0.009411 |
| 9 | 1 | 3.1E-06 | | 9 | 4647 | 0.014085 |
| 10 | 1 | 3.1E-06 | | 10 | 3652 | 0.011069 |
| 11 | 1 | 3.1E-06 | | 11 | 3853 | 0.011679 |
| 12 | 1 | 3.1E-06 | | 12 | 4081 | 0.01237 |
| 13 | 0 | 0 | | 13 | 3055 | 0.00926 |
| 14 | 0 | 0 | | 14 | 4620 | 0.014003 |
| 15 | 0 | 0 | | 15 | 2997 | 0.009084 |
| 16 | 0 | 0 | | 16 | 4455 | 0.013503 |
| 17 | 0 | 0 | | 17 | 2841 | 0.008611 |
| 18 | 0 | 0 | | 18 | 3597 | 0.010903 |
| 19 | 0 | 0 | | 19 | 3446 | 0.010445 |
| 20 | 1 | 3.1E-06 | | 20 | 2882 | 0.008735 |
| 1000 | 1 | 3.1E-06 | | 1000 | 241764 | 0.732796 |

**Table 3**

| ***Consensus Scoring*** | | | | | | |
|---|---|---|---|---|---|---|
| AAYL75 (SARS-CoV2-RBD SSM) | | | | AAYL135 (VEGF SSM) | | |
| 0 | 0 | 0 | | 0% | 0 | 0 |
| 0.05 | 200 | 0.01901 | | 5% | 68 | 0.00189 |
| 0.1 | 843 | 0.080125 | | 10% | 495 | 0.013755 |
| 0.15 | 485 | 0.046098 | | 15% | 24 | 0.000667 |
| 0.2 | 280 | 0.026613 | | 20% | 41 | 0.001139 |
| 0.25 | 252 | 0.023952 | | 25% | 31 | 0.000861 |
| 0.3 | 242 | 0.023002 | | 30% | 36 | 0.001 |
| 0.35 | 218 | 0.02072 | | 35% | 46 | 0.001278 |
| 0.4 | 260 | 0.024712 | | 40% | 110 | 0.003057 |
| 0.45 | 184 | 0.017489 | | 45% | 165 | 0.004585 |
| 0.5 | 143 | 0.013592 | | 50% | 438 | 0.012171 |
| 0.55 | 120 | 0.011406 | | 55% | 366 | 0.01017 |
| 0.6 | 105 | 0.00998 | | 60% | 434 | 0.01206 |
| 0.65 | 158 | 0.015018 | | 65% | 454 | 0.012616 |
| 0.7 | 216 | 0.02053 | | 70% | 679 | 0.018868 |
| 0.75 | 398 | 0.037829 | | 75% | 1012 | 0.028121 |
| 0.8 | 588 | 0.055888 | | 80% | 1636 | 0.045461 |
| 0.85 | 905 | 0.086018 | | 85% | 3000 | 0.083363 |
| 0.9 | 1358 | 0.129075 | | 90% | 6887 | 0.191375 |
| 0.95 | 1627 | 0.154643 | | 95% | 11905 | 0.330814 |
| 1 | 1939 | 0.184298 | | 100% | 8160 | 0.226749 |
| Total | 10521 | | | | 35987 | |

While certain embodiments have been described in terms of the preferred embodiments, it is understood that variations and modifications will occur to those skilled in the art.

## Claims

1. A method comprising:
a. synthesizing a first polynucleotide library comprising multiple first polynucleotides each comprising at least one protein-coding region, and/or encoding at least one region of interest within a protein-coding region, each first polynucleotide independently and optionally comprising one or more non-silent mutations with respect to a reference sequence of the protein-coding region and/or region of interest, at least one of the first polynucleotides encoding the protein-coding region or region of interest comprising at least one silent mutation with respect to the reference sequence, the at least one silent mutation or a combination of silent mutations in a given protein-coding region and/or region of interest providing a first barcode;
b. randomly pairing each first polynucleotide to a unique second randomized barcode nucleotide sequence to produce a second polynucleotide library comprised of second polynucleotides;
c. sequencing the second polynucleotides or at least the first barcode and second randomized barcode thereof; and,
d. mapping each second randomized barcode to a protein-coding region and/or region of interest of a first polynucleotide; wherein a polynucleotide of the second polynucleotide library can be identified by sequencing only the second randomized barcode.

2. The method of claim 1, wherein the second polynucleotides are sequenced by long-read next generation sequencing.

3. The method of any preceding claim, wherein the second barcode is sequenced using short-read next generation sequencing.

4. The method of any preceding claim further comprising determining the identities and relative abundances of polynucleotides encoding one or more protein-coding sequences by sequencing the second randomized barcodes thereof.

5. The method of any preceding claim, wherein the polynucleotides of the second polynucleotide library contain first barcodes and second barcodes that are separated by more than about 300 nucleotides.

6. The method of any preceding claim, wherein the polynucleotides of the second polynucleotide library contain first barcodes and second barcodes separated by less than about 600 nucleotides.

7. The method of any preceding claim, wherein both the first barcode and the second (randomized) barcode contained within the polynucleotides of the second polynucleotide library are sequenced by short-read next-generation sequencing.

8. The method of any preceding claim, wherein both the first and second barcode contained within each polynucleotide of the second polynucleotide library are sequenced by long-read next-generation sequencing.

9. The method of any preceding claim, wherein the first polynucleotide library contains one or more polynucleotides that contain a protein-coding region coding for a protein with a single amino acid mutation with respect to a reference protein sequence, in particular,
wherein one or more polynucleotides from the first polynucleotide library contains a single non silent mutation resulting from a single nucleic acid substitution.

10. The method of any preceding claim, wherein the first barcode comprises three or more silent mutations.

11. The method of any preceding claim, wherein one or more polynucleotides from the first polynucleotide library contains more silent mutations with respect to a reference protein sequence as compared to the number of non-silent nucleic acid mutations with respect to the reference protein sequence.

12. The method of any preceding claim, wherein two or more polynucleotides from the second polynucleotide library contain identical non-silent mutations with respect to a reference protein sequence but different second barcodes such that the two molecules encoding an identical amino acid sequence are identified by sequencing the second barcodes.

13. The method of any preceding claim, wherein one or more protein coding regions in one or more cells are identified by sequencing one or more second barcodes.

14. The method of claim 13 wherein two protein coding regions in one or more cells are identified by sequencing two second barcodes contained within the same cell, in particular, wherein the cell is a yeast diploid cell.

15. The method of claim 14 wherein the yeast diploid cell was produced through the mating of two yeast haploid cells, each comprising one second barcode.

## Patentansprüche

1. Verfahren, umfassend:
a. Synthetisieren einer ersten Polynukleotid-Bibliothek, die eine Mehrzahl erster Polynukleotide umfasst, welche jeweils mindestens eine proteinkodierende Region umfasst und/oder mindestens eine Region von Interesse innerhalb einer proteinkodierenden Region kodieren, wobei jedes erste Polynukleotid unabhängig und optional eine oder mehrere nicht-stille Mutationen in Bezug auf eine Referenzsequenz der proteinkodierenden Region und/oder der Region von Interesse umfasst, wobei mindestens eines der ersten Polynukleotide, welche die proteinkodierende Region oder die Region von Interesse kodieren, mindestens eine stille Mutation in Bezug auf die Referenzsequenz umfasst, wobei die mindestens eine stille Mutation oder eine Kombination von stillen Mutationen in einer gegebenen proteinkodierenden Region und/oder Region von Interesse einen ersten Barcode bereitstellt;
b. zufälliges Paaren jedes ersten Polynukleotids mit einer eindeutigen zweiten randomisierten Barcode-Nukleotidsequenz, um eine zweite Polynukleotid-Bibliothek zu erzeugen, die aus zweiten Polynukleotiden besteht;
c. Sequenzieren der zweiten Polynukleotide oder zumindest des ersten Barcodes und des zweiten randomisierten Barcodes davon; und
d. Zuordnen (Mapping) jedes zweiten randomisierten Barcodes zu einer proteinkodierenden Region und/oder einer Region von Interesse eines ersten Polynukleotids; wobei ein Polynukleotid der zweiten Polynukleotid-Bibliothek durch Sequenzieren nur des zweiten randomisierten Barcodes identifiziert werden kann.

2. Verfahren nach Anspruch 1, wobei die zweiten Polynukleotide mittels Long-Read Next-Generation-Sequencing sequenziert werden.

3. Verfahren nach einem der vorstehenden Ansprüche, wobei der zweite Barcode mittels Short-Read Next-Generation-Sequencing sequenziert wird.

4. Verfahren nach einem der vorstehenden Ansprüche, ferner umfassend das Bestimmen der Identitäten und relativen Häufigkeiten von Polynukleotiden, die eine oder mehrere proteinkodierende Sequenzen kodieren, durch Sequenzieren des zweiten randomisierten Barcodes davon.

5. Verfahren nach einem der vorstehenden Ansprüche, wobei die Polynukleotide der zweiten Polynukleotid-Bibliothek erste Barcodes und zweite Barcodes enthalten, die durch mehr als etwa 300 Nukleotide voneinander getrennt sind.

6. Verfahren nach einem der vorstehenden Ansprüche, wobei die Polynukleotide der zweiten Polynukleotid-Bibliothek erste Barcodes und zweite Barcodes enthalten, die durch weniger als etwa 600 Nukleotide voneinander getrennt sind.

7. Verfahren nach einem der vorstehenden Ansprüche, wobei sowohl der erste Barcode als auch der zweite (randomisierte) Barcode, die in den Polynukleotiden der zweiten Polynukleotid-Bibliothek enthalten sind, mittels Short-Read Next-Generation-Sequencing sequenziert werden.

8. Verfahren nach einem der vorstehenden Ansprüche, wobei sowohl der erste als auch der zweite Barcode, die in jedem Polynukleotid der zweiten Polynukleotid-Bibliothek enthalten sind, mittels Long-Read Next-Generation-Sequencing sequenziert werden.

9. Verfahren nach einem der vorstehenden Ansprüche, wobei die erste Polynukleotid-Bibliothek ein oder mehrere Polynukleotide enthält, die eine proteinkodierende Region enthalten, die für ein Protein mit einer einzelnen Aminosäuremutation in Bezug auf eine Referenzproteinssequenz kodiert, insbesondere
wobei ein oder mehrere Polynukleotide aus der ersten Polynukleotid-Bibliothek eine einzelne nicht-stille Mutation enthalten, die aus einer einzelnen Nukleinsäuresubstitution resultiert.

10. Verfahren nach einem der vorstehenden Ansprüche, wobei der erste Barcode drei oder mehr stille Mutationen umfasst.

11. Verfahren nach einem der vorstehenden Ansprüche, wobei ein oder mehrere Polynukleotide aus der ersten Polynukleotid-Bibliothek, im Vergleich zur Anzahl der nichtstillen Nukleinsäuremutationen in Bezug auf eine Referenzproteinssequenz, mehr stille Mutationen in Bezug auf die Referenzproteinssequenz enthalten.

12. Verfahren nach einem der vorstehenden Ansprüche, wobei zwei oder mehr Polynukleotide aus der zweiten Polynukleotid-Bibliothek identische nicht-stille Mutationen in Bezug auf eine Referenzproteinssequenz, jedoch unterschiedliche zweite Barcodes aufweisen, so dass die zwei Moleküle, die eine identische Aminosäuresequenz kodieren, durch Sequenzieren der zweiten Barcodes identifiziert werden.

13. Verfahren nach einem der vorstehenden Ansprüche, wobei eine oder mehrere proteinkodierende Regionen in einer oder mehreren Zellen durch Sequenzieren eines oder mehrerer zweiter Barcodes identifiziert werden.

14. Verfahren nach Anspruch 13, wobei zwei proteinkodierende Regionen in einer oder mehreren Zellen durch Sequenzieren von zwei in derselben Zelle enthaltenen zweiten Barcodes identifiziert werden, insbesondere wobei die Zelle eine diploide Hefezelle ist.

15. Verfahren nach Anspruch 14, wobei die diploide Hefezelle durch das Paaren zweier haploider Hefezellen erzeugt wurde, die jeweils einen zweiten Barcode aufweisen.

## Revendications

1. Procédé comprenant :
a. la synthèse d'une première bibliothèque de polynucléotides comprenant de multiples premiers polynucléotides comprenant chacun au moins une région de codage de protéines, et/ou codant au moins une région d'intérêt à l'intérieur d'une région de codage de protéines, chaque premier polynucléotide comprenant indépendamment et optionnellement une ou plusieurs mutations non silencieuses par rapport à une séquence de référence de la région de codage de protéines et/ou région d'intérêt, au moins un des premiers polynucléotides codant la région de codage de protéines ou région d'intérêt comprenant au moins une mutation silencieuse par rapport à la séquence de référence, l'au moins une mutation silencieuse ou une combinaison de mutations silencieuses dans une région de codage de protéines et/ou région d'intérêt donnée fournissant un premier code-barres ;
b. l'appariement aléatoire de chaque premier polynucléotide à une deuxième séquence de nucléotides à code-barres randomisé unique pour produire une deuxième bibliothèque de polynucléotides composée de deuxièmes polynucléotides ;
c. le séquençage des deuxièmes polynucléotides ou d'au moins le premier code-barres et deuxième code-barres randomisé de ceux-ci ; et,
d. le mappage de chaque deuxième code-barres randomisé sur une région de codage de protéines et/ou région d'intérêt d'un premier polynucléotide ; dans lequel un polynucléotide de la deuxième bibliothèque de polynucléotides peut être identifié par séquençage uniquement du deuxième code-barres randomisé.

2. Procédé selon la revendication 1, dans lequel les deuxièmes polynucléotides sont séquencés par séquençage de nouvelle génération à lecture longue.

3. Procédé selon une quelconque revendication précédente, dans lequel le deuxième code-barres est séquencé à l'aide du séquençage de nouvelle génération à lecture courte.

4. Procédé selon une quelconque revendication précédente comprenant en outre la détermination des identités et abondances relatives de polynucléotides codant une ou plusieurs séquences de codage de protéines par séquençage des deuxièmes codes-barres randomisés de ceux-ci.

5. Procédé selon une quelconque revendication précédente, dans lequel les polynucléotides de la deuxième bibliothèque de polynucléotides contiennent des premiers codes-barres et des deuxièmes codes-barres qui sont séparés par plus de 300 nucléotides environ.

6. Procédé selon une quelconque revendication précédente, dans lequel les polynucléotides de la deuxième bibliothèque de polynucléotides contiennent des premiers codes-barres et des deuxièmes codes-barres séparés par moins de 600 nucléotides environ.

7. Procédé selon une quelconque revendication précédente, dans lequel le premier code-barres et le deuxième code-barres (randomisé) contenus à l'intérieur des polynucléotides de la deuxième bibliothèque de polynucléotides sont séquencés par séquençage de nouvelle génération à lecture courte.

8. Procédé selon une quelconque revendication précédente, dans lequel les premier et deuxième codes-barres contenus à l'intérieur de chaque polynucléotide de la deuxième bibliothèque de polynucléotides sont séquencés par séquençage de nouvelle génération à lecture longue.

9. Procédé selon une quelconque revendication précédente, dans lequel la première bibliothèque de polynucléotides contient un ou plusieurs polynucléotides qui contiennent une région de codage de protéines codant pour une protéine avec une seule mutation d'acide aminé par rapport à une séquence de protéines de référence, en particulier,
dans lequel un ou plusieurs polynucléotides provenant de la première bibliothèque de polynucléotides contiennent une seule mutation non silencieuse résultant d'une seule substitution d'acide nucléique.

10. Procédé selon une quelconque revendication précédente, dans lequel le premier code-barres comprend trois mutations silencieuses ou plus.

11. Procédé selon une quelconque revendication précédente, dans lequel un ou plusieurs polynucléotides provenant de la première bibliothèque de polynucléotides contiennent plus de mutations silencieuses par rapport à une séquence de protéines de référence comparé au nombre de mutations d'acide nucléique non silencieuses par rapport à la séquence de protéines de référence.

12. Procédé selon une quelconque revendication précédente, dans lequel deux polynucléotides ou plus provenant de la deuxième bibliothèque de polynucléotides contiennent des mutations non silencieuses identiques par rapport à une séquence de protéines de référence mais différents deuxièmes codes-barres de telle sorte que les deux molécules codant une séquence d'acides aminés identique sont identifiées par séquençage des deuxièmes codes-barres.

13. Procédé selon une quelconque revendication précédente, dans lequel une ou plusieurs régions de codage de protéines dans une ou plusieurs cellules sont identifiées par séquençage d'un ou plusieurs deuxièmes codes-barres.

14. Procédé selon la revendication 13 dans lequel deux régions de codage de protéines dans une ou plusieurs cellules sont identifiées par séquençage de deux deuxièmes codes-barres contenus à l'intérieur de la même cellule, en particulier, dans lequel la cellule est une cellule diploïde de levure.

15. Procédé selon la revendication 14 dans lequel la cellule diploïde de levure a été produite par l'accouplement de deux cellules haploïdes de levure, comprenant chacune un deuxième code-barres.
